(19) Europäisches Patentamt
European Patent Office
Office européen des brevets

(11) EP 1 501 471 B1

# (12) EUROPEAN PATENT SPECIFICATION

(45) Date of publication and mention of the grant of the patent:
**07.06.2006 Bulletin 2006/23**

(21) Application number: **03747128.1**

(22) Date of filing: **25.04.2003**

(51) Int Cl.:
***A61K 8/02*** (2006.01) ***A61K 8/04*** (2006.01)
***A61K 8/11*** (2006.01) ***A61K 8/49*** (2006.01)
***A61K 8/86*** (2006.01) ***A61Q 5/00*** (2006.01)
***A61Q 5/02*** (2006.01)

(86) International application number:
**PCT/EP2003/005408**

(87) International publication number:
**WO 2003/090701 (06.11.2003 Gazette 2003/45)**

## (54) USE OF ALPHA-DIALDEHYDES IN THE PRESENCE OF AN AMMONIUM SALT OF A BRÖNSTED ACID FOR DYEING KERATIN FIBRES

VERWENDUNG VON ALPHA-DIALDEHYDEN IN ANWESENHEIT EINES AMMONIUMSALZES EINER BRÖNSTED SÄURE ZUM FÄRBEN VON KERATINFASERN

UTILISATION DE ALPHA-DIALDEHYDES EN PRESENCE D'UN SEL D'AMMONIUM D'UN ACIDE DE BRONSTED POUR LA TEINTURE DE FIBRES KERATINIQUES

(84) Designated Contracting States:
**AT BE BG CH CY CZ DE DK EE ES FI FR GB GR HU IE IT LI LU MC NL PT RO SE SI SK TR**

(30) Priority: **25.04.2002 FR 0205186**
**24.05.2002 US 382632 P**

(43) Date of publication of application:
**02.02.2005 Bulletin 2005/05**

(73) Proprietor: **L'OREAL**
**75008 Paris (FR)**

(72) Inventors:
- **PLOS, Grégory**
  **75011 Paris (FR)**
- **DAUBRESSE, Nicolas**
  **78170 La Celles ST Cloud (FR)**

(74) Representative: **Wattremez, Catherine et al**
**L'OREAL - D.I.P.I.**
**25-29 Quai Aulagnier**
**92600 Asnières (FR)**

(56) References cited:
**WO-A-93/19725**
**DE-A- 10 045 856**
**DE-A- 10 048 922**
**DE-A- 19 820 894**
**DE-A- 19 951 134**
**US-A- 3 871 818**
**US-A- 3 904 357**

- **PATENT ABSTRACTS OF JAPAN vol. 016, no. 114 (C-0921), 23 March 1992 (1992-03-23) & JP 03 287521 A (HOYU CO LTD), 18 December 1991 (1991-12-18)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to the use of α-dialdehydes in the presence of an ammonium salt of a Brönsted acid for dyeing keratin fibres, in particular human keratin fibres such as the hair, to the ready-to-use compositions for dyeing keratin fibres containing at least one α-dialdehyde and at least one ammonium salt of a Brönsted acid, and also to multi-compartment kits for preparing these compositions and for dyeing keratin fibres.

Men and women have for a very long time sought to modify the colour of their hair and in particular to mask grey hair, which is a visible sign of ageing.

In the field of dyeing keratin fibres, in particular human keratin fibres such as the hair, two methods of dyeing are usually distinguished, which each have their advantages and drawbacks:

- *oxidation dyeing* involves the oxidative condensation of small colourless molecules, known as dye precursors, which are generally aromatic diamines, diphenols or aminophenols, in the presence of an oxidizing agent, resulting in the formation of coloured polymer compounds in the keratin fibres.
  The main advantage of oxidation dyeing lies in the longevity of the colorations obtained, in particular in the excellent wash-fastness and the fastness with respect to the surrounding conditions, and in the production of a broad range of shades. However, the chemical dyeing conditions, such as the high pH and a strongly oxidizing medium, result in degradation of the keratin fibres. Moreover, this method of dyeing requires relatively long action times, which is not only inconvenient for the user, but also prolongs the exposure of the hair to the above harmful conditions and consequently aggravates the degradation of the hair;
- *direct dyeing* by adsorption of dyes onto the surface of the keratin fibres and/or by diffusing dyes in the surface layers of these fibres.

The action times are generally fairly short and the mild dyeing conditions preserve the integrity of the keratin fibres, but the colorations obtained by this dyeing method show poor resistance to washing and fade out after shampooing only a few times.

Patent US 3 871 818 describes a process for dyeing the hair with dialdehydes in the presence of at least one nitrogen compound. The said document in particular discloses an example of dyeing using a solution containing 0.1% ortho-phthalaldehyde and 0.1% aqueous ammonia ($NH_4OH$). According to the said document, the dyeing process described gives grey to black colorations depending on the action time. The use of this process in fact leads, on contact with the hair to a green coloration, which is relatively unattractive and unusable.

In the context of its investigations in the field of dyeing keratin fibres, the Applicant has discovered, surprisingly, that it is possible to obtain hair colorations in grey shades, which are extraordinarily fast, by means of a coloured reaction between an α-dialdehyde and an ammonium salt of a Brönsted acid.

Specifically, the Applicant has observed that a certain group of α-dialdehydes described in greater detail hereinbelow reacts with aqueous ammonia in the presence of a Brönsted acid, or with an ammonium salt obtained by neutralizing a Brönsted acid with aqueous ammonia, almost immediately giving a black precipitate, and that this reaction, when performed on keratin fibres, allows these fibres to be dyed in strong grey shades.

In contrast with other coloured reactions used hitherto in the dyeing of human keratin fibres, such as the hair, the reaction between an α-dialdehyde and an ammonium salt, which is the basis of the present invention, gives fastness results, and in particular resistance to shampoo, to light, to sweat and to permanent-waving, which are equivalent if not superior to those usually obtained by oxidation dyeing.

Another advantage of the use of the combination of an α-dialdehyde and an ammonium salt for dyeing keratin fibres lies in the limited degradation of the fibres, since the keratin does not come into contact with powerful oxidizing agents such as aqueous hydrogen peroxide solution usually used for oxidation dyeing. Moreover, the concentrations of aqueous ammonia or of ammonium salt required to obtain satisfactory coloration intensities are less than those usually used in oxidation dyeing.

A subject of the present invention is, consequently, a ready-to-use composition, prepared extemporaneously, for dyeing keratin fibres, comprising, in a cosmetically acceptable medium, at least one α-dialdehyde of formula (I) below and at least one ammonium salt of a Brönsted acid.

A subject of the invention is also a process for dyeing keratin fibres, comprising the application of this ready-to-use composition, prepared extemporaneously, to the keratin fibres, leaving the composition in contact with the keratin fibres for an action time that is sufficient to obtain the desired coloration, and rinsing the keratin fibres.

In addition to this dyeing process comprising a single application step (one-step process or one-stage process), the invention also relates to processes for dyeing keratin fibres in which, rather than applying the ready-to-use composition, the various active components of such a composition are applied separately and successively, for example first the dialdehyde alone, followed by the ammonium salt, the two application steps optionally being separated by a rinsing step. The various methods for performing these two-step processes will be described in greater detail hereinbelow.

A considerable advantage of these two-stage dyeing processes lies in the fact that they allow the hair to be dyed without using a coloured composition, thus making it possible to considerably reduce the risk of staining clothing, the hands or other objects liable to come into contact with the dye composition. The reason for this is that the various compositions, applied separately one after the other, are colourless and will not leave any marks, the colour being developed only *in situ* when the second component is applied.

The α-dialdehydes used in the present invention are compounds of formula (I)

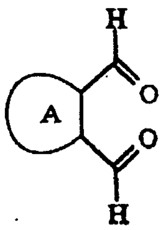

(I)

in which A represents

- an aromatic or non-aromatic carbocyclic, monocyclic or polycyclic group with fused or non-fused rings, containing from 6 to 50 carbon atoms, or
- a 5- to 30-membered aromatic or non-aromatic heterocyclic, monocyclic or polycyclic group with fused or non-fused rings, optionally combined with a carbocyclic, monocyclic or polycyclic group with fused or non-fused rings, containing from 5 to 30 carbon atoms,

the hetero atoms of the heterocyclic group being chosen from nitrogen, sulphur, oxygen and/or phosphorus, and the carbocyclic or heterocyclic groups optionally bearing one or more halo, $C_{1-6}$ alkyl, hydroxyl, $C_{1-6}$ alkoxy, thiol, carboxylic acid, nitro, sulpho or nitrogen-containing heterocyclic substituents.

The Applicant has obtained particularly advantageous results with aromatic α-dialdehyde of formula (I) in which A has the meaning given above and in which at least the ring bearing the two vicinal aldehyde functions is an aromatic ring.

Examples of such preferred compounds that may be mentioned include those in which the two vicinal aldehyde functions are borne by a benzene, naphthalene, anthracene, pyridine or thiophene ring.

Among these compounds, preference is given in particular to ortho-phthalaldehyde or derivatives thereof corresponding to formula (II)

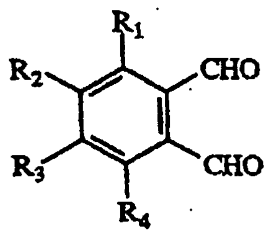

(II)

in which $R^1$, $R^2$, $R^3$ and $R^4$ each independently represent a hydrogen atom or a halo, $C_{1-6}$ alkyl, hydroxyl, $C_{1-6}$ alkoxy, thiol, carboxylic acid, nitro, sulpho or nitrogen-containing non-aromatic heterocyclic substituent.

An aromatic α-dialdehyde that is particularly preferred is unsubstituted ortho-phthalaldehyde, which corresponds to formula (II) above in which $R^1$, $R^2$, $R^3$ and $R^4$ all represent a hydrogen atom.

Other α-dialdehyde that may be used in the present invention and that are commercially available are 4,5-dimethoxy-ortho-phthalaldehyde (CAS 43073-12-7), naphthalene-2,3-dialdehyde (CAS 7149-49-7), anthracene-2,3-dialdehyde (CAS 76197-35-8) and thiophene-2,3-dialdehyde (CAS 932-41-2).

The expression "ammonium salt of a Brönsted acid that may be used in the present invention for dyeing keratin fibres" means any salt obtained by neutralizing a Bronsted acid, i.e. an acid capable of yielding a proton, with aqueous ammonia ($NH_4OH$)

The Brönsted acid may be an organic or mineral compound bearing at least one acid function, such as a carboxylic acid, thiol, hydrogen carbonate, sulphuric acid, sulphonic acid, phosphoric acid or phosphonic acid function.

Examples of ammonium salts of a mineral Brönsted acid that may be mentioned include ammonium sulphate, ammonium hydrogen carbonate and ammonium phosphate.

The ammonium salts of an organic Brönsted acid are, for example, the ammonium salts of carboxylic acids,

ammonium organosulphonates, ammonium organosulphates, ammonium organophosphates, ammonium organo-phosphonates and ammonium thiolates.

Ammonium hydrogen carbonate and ammonium carboxylates, and most particularly ammonium acetate, are particularly preferred in the present invention.

It may be very advantageous in certain cases to form ammonium salts using Brönsted acids whose structure gives them particular physicochemical properties, such as, for example, an ability to absorb visible or UV light, biological activity, surfactant properties or thickening properties.

Examples of such compounds that may be mentioned include ammonium salts of anionic organic dyes comprising at least one acid function, generally a sulphonic acid function, ammonium salts of anionic surfactants comprising at least one acid function, for example ammonium lauryl ether sulphate, ammonium salts of amino acids such as, for example, ammonium glutamate or ammonium aspartate, and polymers comprising acid functions such as, for example, poly(acrylic acid) or poly(methacrylic acid).

The pH of the ready-to-use compositions of the present invention must have a cosmetically acceptable value. It is generally between 3 and 12 and preferably between 6 and 11.

The concentration of $\alpha$-dialdehyde of formula (I) depends on a great many parameters, for example the temperature and the action time, the coloration intensity that it is desired to obtain and the condition of the keratin fibres. The Applicant has obtained satisfactory results with concentrations of $\alpha$-dialdehyde of formula (I) of between 0.01 % and 30% by weight and preferably between 0.05% and 20% by weight, relative to the total weight of the composition.

The concentration of ammonium salt is also preferably between 0.01 % and 30% by weight and in particular between 0.05% and 20% by weight, relative to the total weight of the composition.

The Applicant has obtained particularly advantageous results when the molar ratio of the $\alpha$-dialdehyde to the ammonium salt is between $5 \times 10^{-4}$ and 500, preferably between $5 \times 10^{-2}$ and 50 and in particular between 0.1 and 10.

A subject of the present invention is also a first process for dyeing keratin fibres, in particular the hair, using the ready-to-use compositions described above.

In concrete terms, this process comprises the following successive steps consisting in

- preparing, immediately before use, a ready-to-use composition containing at least one dialdehyde of formula (I) and at least one ammonium salt of a Brönsted acid,
- applying this ready-to-use composition, prepared extemporaneously, to the keratin fibres,
- leaving the composition in contact with the keratin fibres for an action time that is sufficient to obtain the desired coloration, and
- rinsing the keratin fibres.

The ready-to-use composition may be prepared by simple mixing of the various components, packaged separately from each other so as to prevent the coloured reaction between the $\alpha$-dialdehyde and the ammonium salt from taking place before they are placed in contact with the keratin fibres.

Three methods for extemporaneously preparing the ready-to-use composition may be envisaged:

- In the first preparation method, a first composition (composition (a)), which contains at least one $\alpha$-dialdehyde of formula (I), is mixed with a second composition (composition (b)) containing at least one ammonium salt of a Brönsted acid.
- In another method for preparing the ready-to-use composition, a first composition (composition (c)) containing both at least one $\alpha$-dialdehyde of formula (I) and at least one Brönsted acid in acid form, is mixed with a second composition containing aqueous ammonia (composition (d)).
- Needless to say, it is also possible to envisage the preparation by mixing together three different compositions, packaged separately, respectively containing at least one $\alpha$-dialdehyde of formula (I), at least one Bronsted acid and aqueous ammonia.

The ready-to-use composition should be applied to the keratin fibres as quickly as possible, i.e. less than 10 minutes and preferably less than 5 minutes after mixing together the various compositions described above. In order to obtain the excellent fastnesses observed, it is in fact essential, for the process of the present invention, for the coloured reaction to take place in the presence of the keratin fibres.

The action time that is sufficient to obtain the desired coloration is generally between 5 minutes and 1 hour and preferably between 5 and 30 minutes.

As indicated above, the process for dyeing keratin fibres described above requires relatively quick application of the extemporaneously prepared ready-to-use dye composition. During this application, the user risks soiling his clothing, his hands or other surfaces and objects with the coloured composition.

This drawback may be overcome by means of a two-stage dyeing process, which also constitutes a subject of

the present invention.

Such a process involves the successive application of compositions (a) and (b), or of compositions (c) and (d), described above, to keratin fibres, the coloured reaction between the α-dialdehyde and the ammonium salt of a Brönsted acid then starting only when the second composition is applied. In such a two-stage process, the application of the compositions does not require a high speed of execution. Moreover, as indicated above, working in this way considerably reduces the risk of soiling objects other than the keratin fibres.

In concrete terms, the two-stage process for dyeing keratin fibres comprises the following steps consisting in:

- applying to the keratin fibres a first composition (a) containing at least one α-dialdehyde of formula (I) and leaving this composition in contact with the keratin fibres for an action time of between 5 minutes and 1 hour and preferably between 5 and 30 minutes,
- optionally rinsing the keratin fibres with water,
- applying to the keratin fibres a second composition (b) containing at least one ammonium salt of a Bronsted acid and leaving this composition in contact with the keratin fibres for an action time that is sufficient to obtain the desired coloration, and then
- rinsing the keratin fibres.
  Similarly, by using instead of the compositions (a) and (b), the compositions (c) and (d), respectively, another process for dyeing keratin fibres of the present invention comprises the following steps consisting in
- applying to the keratin fibres a first composition (c) containing at least one aromatic α-dialdehyde of formula (I) and at least one Brönsted acid, and leaving this composition in contact with the keratin fibres for an action time of between 5 minutes and 1 hour and preferably between 5 and 30 minutes,
- optionally rinsing the keratin fibres with water,
- applying to the keratin fibres a second composition (d) containing aqueous ammonia and leaving this composition in contact with the keratin fibres for an action time that is sufficient to obtain the desired coloration, and then
- rinsing the keratin fibres.
  In another embodiment, a process for dyeing keratin fibres of the present invention comprises the following steps consisting in
- applying to the keratin fibres a first composition (e) containing at least one aromatic α-dialdehyde of formula (I) and at least aqueous ammonia, and leaving this composition in contact with the keratin fibres for an action time of between 5 minutes and 1 hour and preferably between 5 and 30 minutes,
- optionally rinsing the keratin fibres with water,
- applying to the keratin fibres a second composition (f) containing one Brönsted acid and leaving this composition in contact with the keratin fibres for an action time that is sufficient to obtain the desired coloration, and then
- rinsing the keratin fibres.

The duration of the second action time required to obtain the desired coloration is generally within the same limit values as that of the first action time, i.e. between 5 minutes and one hour and preferably between 5 and 30 minutes.

In one variant of the two-stage process for dyeing keratin fibres described above, the order of application of compositions (a) and (b), or of compositions (c) and (d), or of compositions (e) and (f) is reversed.

The coloured reaction between the α-dialdehyde and the ammonium salt which is the basis of the processes for dyeing keratin fibres of the present invention may be accelerated by means of applying heat, for example using radiation, for example infrared radiation, or hot, dry or wet air, derived, for example, from a hairdryer or a steam generator. This supply of heat for the action time is generally performed so as to increase the temperature to a value of not more than 80°C and preferably not more than 60°C.

According to a variant of the process for dyeing keratin fibres, a post treatment can be made, consisting in applying an acidic solution and leaving this solution in contact with the keratin fibres, and then rinsing the keratin fibres.

The acidic solution, which is preferably an aqueous solution, has a pH of at most 7, and more particularly comprised between 3 and 7. The pH can be regulated by any conventional means.

The acid can be advantageously a Brönsted acid.

The duration of this post-treatment is generally comprised between 10 seconds and an hour, preferably between 10 seconds and 30 minutes.

It is noted that the post-treatment can be made by using an acidic shampoo, a lotion, a spray or a gel.

A subject of the present invention is also dyeing kits for performing the one-stage or two-stage processes for dyeing keratin fibres described above. These kits contain the various reagents involved in the coloured reaction, packaged in separate compartments or containers.

A first dyeing kit comprises at least two compartments, at least one of these compartments containing at least one α-dialdehyde of formula (I) (composition (a)) and at least one other compartment containing at least one ammonium salt of a Brönsted acid (composition (b)).

Similarly, a second kit for dyeing keratin fibres comprises at least two compartments, at least one of these compartments containing at least one α-dialdehyde of formula (I) and at least one Brönsted acid (composition (c)) and at least one other compartment containing aqueous ammonia (composition (d)).

Finally, it may also be envisaged to package the three reagents involved in the coloured reaction that is the basis of the present invention in three separate compartments. Such a dyeing kit contains at least three compartments, at least a first containing an α-dialdehyde of formula (I), at least a second containing a Brönsted acid and at least a third containing aqueous ammonia.

In all the dyeing kits described above, the α-dialdehyde, the ammonium salt of a Brönsted acid, are each preferably present in a proportion of from 0.01% to 30% by weight and more particularly in a proportion of from 0.05% to 20% by weight, relative to the total weight of the compositions contained in the various compartments.

Moreover, when the Brönsted acid and the aqueous ammonia are present in separate compartments, they are each present in amounts such as the amount of the ammonium salt of a Brönsted acid in the ready-to-use composition represents 0.01 % to 30% by weight and more particularly 0.05% to 20% by weight, relative to the total weight of the said composition. More particularly, the Brönsted acid and the aqueous ammonia are each preferably present in a proportion of from 0.01% to 30% by weight and more particularly in a proportion of from 0.05% to 20% by weight, relative to the total weight of the compositions contained in the various compartments.

The ready-to-use dye compositions and the compositions (a), (b), (c) and (d) above may also contain other dyes, cosmetic active principles or formulation adjuvants that are well known in cosmetics and in particular in the field of dyeing keratin fibres.

The dyes may be chosen, for example, from oxidation dye precursors (bases and couplers) and direct dyes, in particular cationic direct dyes and dyes of natural origin.

The cosmetic active principles may be chosen from vitamins, saccharides, oligosaccharides, hydrolysed or non-hydrolysed, modified or unmodified polysaccharides, amino acids, oligopeptides, peptides, hydrolysed or non-hydrolysed, modified or unmodified proteins, polyamino acids, enzymes, branched or unbranched fatty acids and alcohols, animal, plant or mineral waxes, ceramides and pseudoceramides, hydroxylated organic acids, UV-screening agents, antioxidants, free-radical scavengers, chelating agents, antidandruff agents, seborrhoea regulators, calmants, cationic surfactants, cationic polymers, amphoteric polymers, organomodified or non-organomodified silicones, mineral, plant or animal oils, polyisobutenes and poly(α-olefins), fatty esters, anionic polymers in dissolved or dispersed form, nonionic polymers in dissolved or dispersed form, reducing agents and pigments.

The formulation adjuvants are chosen, for example, from thickeners, agents for adjusting and fixing the pH, preserving agents, antifoams, fragrances and non-cationic surfactants.

The present invention is illustrated below with the aid of the implementation examples.

**Example 1**

<u>One-stage dyeing process</u>

The ready-to-use dye composition is prepared, immediately before application, by mixing together the following ingredients:

| | |
|---|---|
| ortho-phthalaldehyde | 0.4% by weight |
| ammonium acetate | 1.3% by weight |
| distilled water | qs 100% |

This composition is applied to natural and permanent-waved hair containing 90% white hairs, and is left on the hair for 5 minutes. At the end of the action time, the hair is rinsed with water and washed with a standard shampoo.

The dyeing intensity is evaluated by colorimetry according to the CIELAB system using a Minolta CM2002 colorimeter (illuminant D65, observation angle: 10°, specular components excluded).

The CIELAB notation system defines a colorimetric space in which each colour is defined by 3 parameters (L*, a* and b*):

- the parameter L* reflects the *lightness* of the colour, the value of L* being equal to 0 for black and equal to 1 for absolute white. The higher the value of L*, the less intense the coloration;
- the parameters a* and b* define the *chromaticity*: a* corresponds to the axis of the green-red antagonistic pair, and b* to the axis of the blue-yellow antagonist pair.

Table 1 below shows the parameters L*, a* and b* of locks of natural hair and permanent-waved hair, before

and after the rise in colour.

**Table 1** Colorimetric results

| | **Natural grey hair** | | | **Permanent-waved grey hair** | | |
|---|---|---|---|---|---|---|
| | **L*** | **a*** | **b*** | **L*** | **a*** | **b*** |
| rise | 54.9 | 0.8 | 12.0 | 52.4 | 0.9 | 12.3 |
| Before After rise | 34.5 | -1.3 | 2.5 | 30.0 | -0.6 | 1.3 |

The coloration obtained is grey and relatively unselective.

The locks of natural and permanent-waved hair, dyed as described above, are then subjected to a series of 24 shampoo washes and the parameters L*, a* and b* of the CIELAB colorimetric space are measured, respectively, after 6, 12 and 24 shampoo washes, from which measurements is calculated the value of ΔE defined by the equation below:

$$\Delta E = \sqrt{(L^*_{final} - L^*_{initial})^2 + (a^*_{final} - a^*_{initial})^2 + (b^*_{final} - b^*_{initial})^2}$$

ΔE reflects the overall colour change. Its value is proportionately greater the larger the colour change.

Table 2 below shows the overall colour changes (ΔE) measured after 6, 12 and 24 shampoo washes of natural and permanent-waved dyed hair.

**Table 2** Overall colour change (ΔE) after several shampoo washes

| | **Natural hair** | **Permanent-waved hair** |
|---|---|---|
| 6 shampoo washes | 2.3 | 0.5 |
| 12 shampoo washes | 1.7 | 0.9 |
| 24 shampoo washes | 4.3 | 2.2 |

These results show that the colorations obtained show excellent resistance to shampooing. The overall colour change is particularly low for permanent-waved hair.

**Example 2**

Two-stage dyeing process

Compositions A and B below are prepared:

| | **Composition A** | **Composition B** |
|---|---|---|
| Ortho-phthalaldehyde | 0.4% | - |
| Ammonium sulphate | - | 10% |
| Distilled water | qs 100% | qs 100% |

Compositions A and B are successively applied to locks of natural hair and to locks of permanent-waved hair, containing 90% white hairs, with action times and in the order specified in Table 3 below. Between the two applications, the locks are rinsed briefly with water. At the end of the second action time, the hair is rinsed with water and washed with a standard shampoo.

The colorimetric results obtained on natural and permanent-waved hair are collated in Table 3 below.

**Table 3**

| | Action time | Nature of the hair | L* | a* | b* |
|---|---|---|---|---|---|
| Before rise | - | Natural | 54.9 | 0.8 | 12.0 |
| Before rise | - | Permanent-waved | 52.4 | 0.9 | 12.3 |
| Composition B | 15 min. | Natural | 35.28 | -0.37 | 2.60 |
| Composition A | 15 min. | Permanent-waved | 32.38 | 0.96 | 1.91 |
| Composition A | 15 min. | Natural | 44.14 | 1.22 | 6.56 |
| Composition B | 15 min. | Permanent-waved | 39.43 | 1.70 | 4.53 |
| Composition B | 5 min. | Natural | 37.35 | 0.25 | 3.16 |
| Composition A | 5 min. | Permanent-waved | 36.96 | 1.94 | 3.62 |
| Composition A | 5 min. | Natural | 44.07 | -0.28 | 5.50 |
| Composition B | 5 min. | Permanent-waved | 37.64 | 0.44 | 4.04 |

These results show that the two-stage process with intermediate rinsing with water also makes it possible to obtain intense grey colorations.

**Example 3**

Fastnesses of the colorations obtained

Natural hair containing 90% white hairs were dyed according to the one-stage process using a ready-to-use composition according to the invention containing 0.4% by weight of ortho-phthalaldehyde and 1.3% by weight of ammonium acetate, prepared immediately before application to the locks of hair.
The fastness of the colorations with respect to light, permanent-waving and perspiration was evaluated on permanent-waved hair by subjecting the dyed locks to the following conditions:

- resistance to light: Suntest 18 hours
- resistance to perspiration: 48 hours synthetic sweat, pH 3.2, temperature: 32°C
- resistance to permanent-waving: Dulcia Tonica No. 2

The parameters L*, a* and b* are measured before and after exposing the dyed hair to the conditions indicated above, and the overall colour change ($\Delta E_{resistance}$) was deduced therefrom by means of the equation indicated above.
Table 4 below shows the colour change observed after the resistance test ($\Delta E_{resistance}$). The larger this change, the smaller the resistance (fastness) of the colorations obtained.
The results according to the invention are compared with those obtained by using for the hair dyeing a dye solution containing 0.4% by weight of ortho-phthalaldehyde and 0.4% by weight of aqueous ammonia in water (comparative example).

**Table 4** Coloration fastnesses - comparison with the prior art

| | | $\Delta E_{resistance}$ |
|---|---|---|
| Resistance to light | Example according to the invention | 1.0 |
| | Comparative example | 5.4 |
| Resistance to permanent-waving | Example according to the invention | 1.0 |
| | Comparative example | 3.4 |
| Resistance to perspiration | Example according to the invention | 1.3 |
| | Comparative example | 3.9 |

These results demonstrate the importance of the Brönsted acid on the fastnesses of the colorations obtained. Specifically, it may be seen that the fastnesses of the colorations obtained with a dye composition according to the

invention (α-dialdehyde + ammonium salt) are greater than those of the colorations obtained with a dye composition simply containing an α-dialdehyde and aqueous ammonia.

## Claims

**1.** Extemporaneously prepared ready-to-use composition for dyeing keratin fibres, comprising, in a cosmetically acceptable medium,

(A) at least one α-dialdehyde of formula (I)

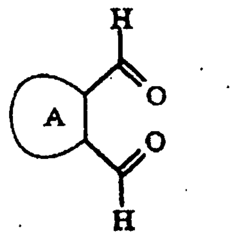

(I)

in which A represents

- an aromatic or non-aromatic carbocyclic, monocyclic or polycyclic group with fused or non-fused rings, containing from 6 to 50 carbon atoms, or
- a 5- to 30-membered aromatic or non-aromatic heterocyclic, monocyclic or polycyclic group with fused or non-fused rings, optionally combined with a carbocyclic, monocyclic or polycyclic group with fused or non-fused rings, containing from 5 to 30 carbon atoms,

the hetero atoms of the heterocyclic group being chosen from nitrogen, sulphur, oxygen and/or phosphorus, and the carbocyclic or heterocyclic groups optionally bearing one or more halo, $C_{1-6}$ alkyl, hydroxyl, $C_{1-6}$ alkoxy, thiol, carboxylic acid, nitro, sulpho or nitrogen-containing heterocyclic substituents, and
(B) at least one ammonium salt of a Brönsted acid.

**2.** Composition according to Claim 1, **characterized in that** A represents a carbocyclic or heterocyclic group as defined in Claim 1, in which at least the ring bearing the two vicinal aldehyde functions is an aromatic ring.

**3.** Composition according to Claim 2, **characterized in that** the α-dialdehyde of formula (I) is an ortho-phthalaldehyde of formula (II)

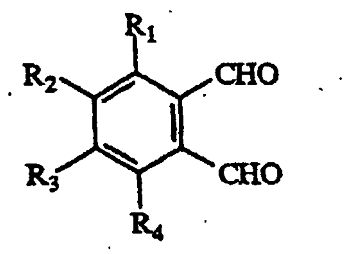

(II)

in which $R^1$, $R^2$, $R^3$ and $R^4$ each independently represent a hydrogen atom or a halo, $C_{1-6}$ alkyl, hydroxyl, $C_{1-6}$ alkoxy, thiol, carboxylic acid, nitro, sulpho or nitrogen-containing heterocyclic substituent.

**4.** Composition according to Claim 3, **characterized in that** $R^1$, $R^2$, $R^3$ and $R^4$ all represent a hydrogen atom.

**5.** Composition according to Claim 2, **characterized in that** the α-dialdehyde is chosen from 4,5-dimethoxy-ortho-phthalaldehyde, naphthalene-2,3-dialdehyde, anthracene-2,3-dialdehyde and thiophene-2,3-dialdehyde.

**6.** Composition according to any one of Claims 1 to 5, **characterized in that** the ammonium salt(s) of a mineral Bronsted acid is(are) chosen from ammonium sulphate, ammonium hydrogen carbonate and ammonium phosphate.

**7.** Composition according to any one of Claims 1 to 5, **characterized in that** the ammonium salt(s) of an organic Brönsted acid is(are) chosen from the ammonium salts of carboxylic acids, ammonium organosulphonates, ammonium organosulphates, ammonium organophosphates, ammonium organophosphonates and ammonium thiolates.

**8.** Composition according to Claim 7, **characterized in that** the organic ammonium salt(s) is(are) chosen from ammonium hydrogen carbonate and ammonium carboxylates.

**9.** Composition according to Claim 8, **characterized in that** the organic ammonium salt is ammonium acetate.

**10.** Composition according to Claim 7, **characterized in that** the organic ammonium salt(s) is(are) chosen from ammonium salts of anionic organic dyes comprising at least one acid function, ammonium salts of anionic surfactants comprising at least one acid function, ammonium salts of amino acids and polymers comprising acid functions.

**11.** Composition according to any one of the preceding claims, **characterized in that** it has a pH of between 3 and 12 and preferably between 6 and 11.

**12.** Composition according to any one of the preceding claims, **characterized in that** the concentration of $\alpha$-dialdehyde of formula (I) is between 0.01 % and 30% by weight and preferably between 0.05% and 20% by weight, relative to the total weight of the composition.

**13.** Composition according to any one of the preceding claims, **characterized in that** the concentration of ammonium salt of a Bronsted acid is between 0.01% and 30% by weight and preferably between 0.05% and 20% by weight, relative to the total weight of the composition.

**14.** Process for dyeing keratin fibres, comprising the following steps consisting in

- preparing, immediately before use, a ready-to-use composition according to any one of the preceding claims,
- applying this ready-to-use composition, prepared extemporaneously, to the keratin fibres,
- leaving the composition in contact with the keratin fibres for an action time that is sufficient to obtain the desired coloration, and
- rinsing the keratin fibres.

**15.** Process for dyeing keratin fibres according to Claim 14, **characterized in that** the ready-to-use composition is prepared by mixing together a first composition (a) containing at least one $\alpha$-dialdehyde of formula (I) and a second composition (b) containing at least one ammonium salt of a Bronsted acid.

**16.** Process according to Claim 14, **characterized in that** the ready-to-use composition is prepared by mixing together a first composition (c) containing at least one $\alpha$-dialdehyde of formula (I) and at least one Bronsted acid, and a second composition (d) containing aqueous ammonia.

**17.** Process according to Claim 14, **characterized in that** the ready-to-use composition is prepared by mixing together three compositions containing, respectively, at least one $\alpha$-dialdehyde of formula (I), at least one Brönsted acid and aqueous ammonia.

**18.** Process according to any one of Claims 14 to 17, **characterized in that** the ready-to-use composition is left in contact with the keratin fibres for a period of between 5 minutes and 1 hour and preferably between 5 and 30 minutes.

**19.** Process for dyeing keratin fibres, comprising the following steps consisting in

- applying to the keratin fibres a first composition (a) containing at least one $\alpha$-dialdehyde of formula (I) and leaving this composition in contact with the keratin fibres for an action time of between 5 minutes and 1 hour,
- optionally rinsing the keratin fibres with water,
- applying to the keratin fibres a second composition (b) containing at least one ammonium salt of a Brönsted acid and leaving this composition in contact with the keratin fibres for an action time that is sufficient to obtain the desired coloration, and then
- rinsing the keratin fibres.

**20.** Process according to Claim 19, **characterized in that** the order of application of compositions (a) and (b) is reversed.

**21.** Process for dyeing keratin fibres, comprising the following steps consisting in

- applying to the keratin fibres a first composition (c) containing at least one aromatic α-dialdehyde of formula (I) and at least one Brönsted acid, and leaving this composition in contact with the keratin fibres for an action time of between 5 minutes and 1 hour,
- optionally rinsing the keratin fibres with water,
- applying to the keratin fibres a second composition (d) containing aqueous ammonia and leaving this composition in contact with the keratin fibres for an action time that is sufficient to obtain the desired coloration, and then
- rinsing the keratin fibres.

**22.** Process according to Claim 21, **characterized in that** the order of application of compositions (c) and (d) is reversed.

**23.** Process for dyeing keratin fibres, comprising the following steps consisting in

- applying to the keratin fibres a first composition (e) containing at least one aromatic α-dialdehyde of formula (I) and at least aqueous ammonia, and leaving this composition in contact with the keratin fibres for an action time of between 5 minutes and 1 hour and preferably between 5 and 30 minutes,
- optionally rinsing the keratin fibres with water,
- applying to the keratin fibres a second composition (f) containing one Brönsted acid and leaving this composition in contact with the keratin fibres for an action time that is sufficient to obtain the desired coloration, and then
- rinsing the keratin fibres.

**24.** Process according to Claim 23, **characterized in that** the order of application of compositions (e) and (f) is reversed.

**25.** Process according to any one of Claims 14 to 24, **characterized in that** heat is applied during the action time so as to increase the temperature to a value of not more than 80°C and preferably not more than 60°C.

**26.** Process according to any one of Claims 14 to 25, **characterized in that** a post treatment is made, consisting in applying an acidic solution and leaving this solution in contact with the keratin fibres, and then rinsing the keratin fibres.

**27.** Kit for dyeing keratin fibres, comprising at least two compartments, at least one of these compartments containing at least one α-dialdehyde of formula (I) (composition (a)) and at least one other compartment containing at least one ammonium salt of a Brönsted acid (composition (b)).

**28.** Kit for dyeing keratin fibres, comprising at least two compartments, at least one of these compartments containing at least one α-dialdehyde of formula (I) and at least one Brönsted acid (composition (c)) and at least one other compartment containing aqueous ammonia (composition (d)).

**29.** Kit for dyeing keratin fibres, comprising at least two compartments, at least one of these compartments containing at least one α-dialdehyde of formula (I) and aqueous ammonia (composition (e)) and at least one other compartment containing at least one Brönsted acid (composition (f)).

**30.** Kit for dyeing keratin fibres, containing at least three compartments, at least a first containing an α-dialdehyde of formula (I), at least a second containing a Brönsted acid and at least a third containing aqueous ammonia.

## Patentansprüche

**1.** Bedarfsgemäß hergestellte gebrauchsfertige Zusammensetzung zum Färben von Keratinfasern, die in einem kosmetisch akzeptablen Medium enthält:

(A) mindestens einen α-Dialdehyden der Formel (I)

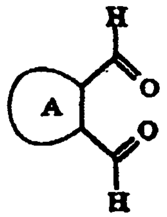 (I)

in welcher A bedeutet:

- eine aromatische oder nichtaromatische, carbocyclische, monocyclische oder polycyclische Gruppe mit kondensierten oder nichtkondensierten Ringen, die 6 bis 50 Kohlenstoffatome enthalten, oder
- eine 5- bis 30-gliedrige, aromatische oder nichtaromatische, heterocyclische, monocyclische oder polycyclische Gruppe mit kondensierten oder nichtkondensierten Ringen, die gegebenenfalls mit einer carbocyclischen, monocyclischen oder polycyclischen Gruppe mit kondensierten oder nichtkondensierten Ringen mit 5 bis 30 Kohlenstoffatomen kombiniert sind,

wobei die Heteroatome der heterocyclischen Gruppe unter Stickstoff, Schwefel, Sauerstoff und/oder Phosphor ausgewählt sind, die carbocyclischen oder heterocyclischen Gruppen wahlweise ein oder mehrere Halogen-, $C_{1-6}$-Alkyl-, Hydroxyl-, $C_{1-6}$-Alkoxy-, Thiol-, Carbonsäure-, Nitro-, Sulfo- oder Stickstoff-haltige heterocyclische Substituenten aufweisen, und
(B) mindestens ein Ammoniumsalz einer Brönsted-Säure.

2. Zusammensetzung nach Anspruch 1, **dadurch gekennzeichnet, dass** A eine carbocyclische oder heterocyclische Gruppe gemäß Anspruch 1 darstellt, in der zumindest der Ring, der die zwei benachbarten Aldehydfunktionen trägt, ein aromatischer Ring ist.

3. Zusammensetzung nach Anspruch 2, **dadurch gekennzeichnet, dass** der $\alpha$-Dialdehyd der Formel (I) ein ortho-Phthalaldehyd der Formel (II) ist,

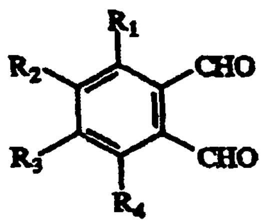 (II)

in welcher die Reste $R^1$, $R^2$, $R^3$ und $R^4$ jeweils unabhängig voneinander ein Wasserstoffatom oder einen Halogen-, $C_{1-6}$-Alkyl-, Hydroxyl-, $C_{1-6}$-Alkoxy-, Thiol-, Carbonsäure-, Nitro-, Sulfo- oder Stickstoff-haltigen heterocyclischen Substituenten bedeuten.

4. Zusammensetzung nach Anspruch 3, **dadurch gekennzeichnet, dass** die Gruppen $R^1$, $R^2$, $R^3$ und $R^4$ ein Wasserstoffatom bedeuten.

5. Zusammensetzung nach Anspruch 2, **dadurch gekennzeichnet, dass** der $\alpha$-Dialdehyd unter 4,5-Dimethoxy-orthophthalaldehyd, Naphthalin-2,3-dialdehyd, Anthracen-2,3-dialdehyd oder Thiophen-2,3-dialdehyd ausgewählt ist.

6. Zusammensetzung nach einem der Ansprüche 1 bis 5, **dadurch gekennzeichnet, dass** das/die Ammoniumsalz (e) einer anorganischen Brönsted-Säure unter Ammoniumsulfat, Ammoniumhydrogencarbonat und Ammoniumphosphat ausgewählt ist/ sind.

7. Zusammensetzung nach einem der Ansprüche 1 bis 5, **dadurch gekennzeichnet, dass** das/die Ammoniumsalz (e) einer anorganischen Brönsted-Säure unter den Ammoniumsalzen von Carbonsäuren, Ammoniumorganosulfonaten, Ammoniumorganosulfaten, Ammoniumorganophosphaten, Ammoniumorganophosphonaten und Ammoniumthiolaten ausgewählt ist/ sind.

**8.** Zusammensetzung nach Anspruch 7, **dadurch gekennzeichnet, dass** das/die organische(n) Ammoniumsalz(e) unter Ammoniumhydrogencarbonat und Ammoniumcarboxylaten ausgewählt ist/ sind.

**9.** Zusammensetzung nach Anspruch 8, **dadurch gekennzeichnet, dass** es sich bei dem organischen Ammoniumsalz um Ammoniumacetat handelt.

**10.** Zusammensetzung nach Anspruch 7, **dadurch gekennzeichnet, dass** das/die organische(n) Ammoniumsalz(e) unter Ammoniumsalzen von anionischen organischen Färbemitteln, die mindestens eine Säurefunktion enthalten, oder Ammoniumsalzen von anionischen grenzflächenaktiven Stoffen, die mindestens eine Säurefunktion enthalten, oder Ammoniumsalzen von Aminosäuren und Polymeren, die eine Säurefunktion enthalten, ausgewählt ist/ sind.

**11.** Zusammensetzung nach einem der vorhergehenden Ansprüche, **dadurch gekennzeichnet, dass** der pH-Wert im Bereich von 3 bis 5 und vorzugsweise 6 bis 11 liegt.

**12.** Zusammensetzung nach einem der vorhergehenden Ansprüche, **dadurch gekennzeichnet, dass** die Konzentration des $\alpha$-Dialdehyds der Formel (I) im Bereich von 0,01 bis 30 Gew.-% und vorzugsweise im Bereich von 0,05 bis 20 Gew.-% liegt, bezogen auf das Gesamtgewicht der Zusammensetzung.

**13.** Zusammensetzung nach einem der vorhergehenden Ansprüche, **dadurch gekennzeichnet, dass** die Konzentration des Ammoniumsalzes einer Brönsted-Säure im Bereich von 0,01 bis 30 Gew.-% und vorzugsweise im Bereich von 0,05 bis 20 Gew.-% liegt, bezogen auf das Gesamtgewicht der Zusammensetzung.

**14.** Verfahren zum Färben von Keratinfasern, das die folgenden Schritte umfasst:

- Herstellen einer gebrauchsfertigen Zusammensetzung nach einem der vorangehenden Ansprüche, unmittelbar vor dem Gebrauch,
- Auftragen dieser gebrauchsfertigen, bedarfsgemäß hergestellten Zusammensetzung auf die Keratinfasern,
- in Kontaktbelassen der Zusammensetzung mit den Keratinfasern während einer Einwirkzeit, die ausreicht, um die gewünschte Färbung zu erhalten, und
- Spülen der Keratinfasern.

**15.** Verfahren zum Färben von Keratinfasern nach Anspruch 14, **dadurch gekennzeichnet, dass** die gebrauchsfertige Zusammensetzung durch Mischen einer ersten Zusammensetzung (a), die mindestens einen $\alpha$-Dialdehyden der Formel (I) enthält, mit einer zweiten Zusammensetzung (b) mit mindestens einem Ammoniumsalz einer Brönsted-Säure hergestellt wird.

**16.** Verfahren nach Anspruch 14, **dadurch gekennzeichnet, dass** die gebrauchsfertige Zusammensetzung durch Mischen einer ersten Zusammensetzung (c) mit mindestens einem $\alpha$-Dialdehyden der Formel (I) und mindestens einer Brönsted-Säure, mit einer zweiten Zusammensetzung (d), die eine wässrige Ammoniaklösung enthält, hergestellt wird.

**17.** Verfahren nach Anspruch 14, **dadurch gekennzeichnet, dass** die gebrauchsfertige Zusammensetzung durch Mischen dreier Zusammensetzungen hergestellt wird, die mindestens einen $\alpha$-Dialdehyden der Formel (I), mindestens eine Brönsted-Säure bzw. eine wässrige Ammoniaklösung enthalten.

**18.** Verfahren nach einem der Ansprüche 14 bis 17, **dadurch gekennzeichnet, dass** die gebrauchsfertige Zusammensetzung für einen Zeitraum von 5 Minuten bis 1 Stunde und vorzugsweise von 5 bis 30 Minuten mit den Keratinfasern in Kontakt belassen wird.

**19.** Verfahren zum Färben von Keratinfasern, das die folgenden Schritte umfasst:

- Auftragen einer ersten Zusammensetzung (a) auf die Keratinfasern, die mindestens einen $\alpha$-Dialdehyden der Formel (I) enthält, und in Kontaktbelassen dieser Zusammensetzung mit den Keratinfasern während einer Einwirkzeit von 5 Minuten bis 1 Stunde,
- gegebenenfalls Spülen der Keratinfasern mit Wasser,

• Auftragen einer zweiten Zusammensetzung (b) auf die Keratinfasern, die mindestens ein Ammoniumsalz einer Brönsted-Säure enthält, und in Kontaktbelassen dieser Zusammensetzung mit den Keratinfasern während einer Einwirkzeit, die ausreicht, um die erwünschte Färbung zu erhalten, und
• Spülen der Keratinfasern.

**20.** Verfahren nach Anspruch 19, **dadurch gekennzeichnet, dass** das Auftragen der Zusammensetzungen (a) und (b) in umgekehrter Reihenfolge erfolgt.

**21.** Verfahren zum Färben von Keratinfasern mit den folgenden Schritten:

• Auftragen einer ersten Zusammensetzung (c) auf die Keratinfasern, die mindestens einen aromatischen α-Dialdehyden der Formel (I) und mindestens eine Brönsted-Säure enthält, und in Kontaktbelassen dieser Zusammensetzung mit den Keratinfasern während einer Einwirkzeit von 5 Minuten bis 1 Stunde,
• gegebenenfalls Spülen der Keratinfasern mit Wasser,
• Auftragen einer zweiten Zusammensetzung (d) auf die Keratinfasern, die eine wässrige Ammoniaklösung enthält, in Kontaktbelassen dieser Zusammensetzung mit den Keratinfasern während einer Einwirkzeit, die ausreicht, um die gewünschte Färbung zu erhalten, und
• Spülen der Keratinfasern.

**22.** Verfahren nach Anspruch 21, **dadurch gekennzeichnet, dass** das Auftragen der Zusammensetzungen (c) und (d) in umgekehrter Reihenfolge erfolgt.

**23.** Verfahren zum Färben von Keratinfasern, das die folgenden Schritte umfasst:

• Auftragen einer ersten Zusammensetzung (e) auf die Keratinfasern, die mindestens einen aromatischen α-Dialdehyden der Formel (I) und mindestens eine wässrige Ammoniaklösung enthält, und in Kontaktbelassen dieser Zusammensetzung mit den Keratinfasern während einer Einwirkzeit von 5 Minuten bis 1 Stunde und vorzugsweise 5 Minuten bis 30 Minuten,
• gegebenenfalls Spülen dieser Keratinfasern mit Wasser,
• Auftragen einer zweiten Zusammensetzung (f) auf die Keratinfasern, die eine Brönsted-Säure enthält, und in Kontaktbelassen dieser Zusammensetzung mit den Keratinfasern während einer Einwirkzeit, die ausreicht, um die gewünschte Färbung zu erhalten, und
• Spülen der Keratinfasern.

**24.** Verfahren nach Anspruch 23, **dadurch gekennzeichnet, dass** das Auftragen der Zusammensetzungen (e) und (f) in umgekehrter Reihenfolge erfolgt.

**25.** Verfahren nach einem der Ansprüche 14 bis 24, **dadurch gekennzeichnet, dass** während der Einwirkzeit Wärme zugeführt wird, um die Temperatur auf einen Wert von nicht mehr als 80 °C und vorzugsweise nicht mehr als 60 °C zu erhöhen.

**26.** Verfahren nach einem der Ansprüche 14 bis 25, **dadurch gekennzeichnet, dass** eine Nachbehandlung vorgenommen wird, die darin besteht, eine saure Lösung aufzutragen und diese Lösung in Kontakt mit den Keratinfasern zu lassen, und anschließend die Keratinfasern zu spülen.

**27.** Vorrichtung zum Färben von Keratinfasern, die mindestens zwei Abteilungen enthält, wobei mindestens eine dieser Abteilungen mindestens einen α-Dialdehyden der Formel (I) (Zusammensetzung (a)), und mindestens eine andere Abteilung mindestens ein Ammoniumsalz einer Brönsted-Säure (Zusammensetzung (b)) enthält.

**28.** Vorrichtung zum Färben von Keratinfasern mit mindestens zwei Abteilungen, wovon mindestens eine dieser Abteilungen mindestens einen α-Dialdehyden der Formel (I) und mindestens eine Brönsted-Säure enthält (Zusammensetzung (c)), und mindestens eine andere Abteilung, die eine wässrige Ammoniaklösung (Zusammensetzung (d)) enthält.

**29.** Vorrichtung zum Färben von Keratinfasern mit mindestens zwei Abteilungen, wovon mindestens eine dieser Abteilungen mindestens einen α-Dialdehyden der Formel (I) und eine wässrige Ammoniaklösung enthält (Zusammensetzung (e)), und mindestens eine andere Abteilung, die mindestens eine Brönsted-Säure (Zusammensetzung (f)) enthält.

**30.** Vorrichtung zum Färben von Keratinfasern, in der mindestens drei Abteilungen enthalten sind, nämlich mindestens eine mit einem $\alpha$-Dialdehyden der Formel (I), mindestens eine zweite mit einer Brönsted-Säure und mindestens eine dritte mit einer wässrigen Ammoniaklösung.

**Revendications**

**1.** Composition de teinture des fibres kératinlques prête à l'emploi, préparée extemporanément, comprenant, dans un milieu cosmétiquement acceptable,

(A) au moins un $\alpha$-dialdéhyde de formule (I)

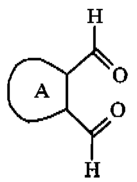

(I)

où A représente

- un groupe carbocyclique, monocyclique ou polycyclique à cycles condensés ou non-condensés, aromatique ou non aromatique, comprenant de 6 à 50 atomes de carbone, ou
- un groupe hétérocyclique, monocyclique ou polycyclique à cycles condensés ou non-condensés, aromatique ou non-aromatique, comprenant de 5 à 30 chaînons, éventuellement associé à un groupe carbocyclique, monocyclique ou polycyclique à cycles condensés ou non-condensés, comprenant de 5 à 30 atomes de carbone,

les hétéroatomes du groupe hétérocyclique étant choisis parmi l'azote, le soufre, l'oxygène et/ou le phosphore, et les groupes carbocycliques ou hétérocycliques portant éventuellement un ou plusieurs substituants halogéno, alkyle en $C_{1-6}$, hydroxyle, alcoxy en $C_{1-6}$, thiol, acide carboxylique, nitro, sulfo ou hétérocyclique azoté, et
(B) au moins un sel d'ammonium d'un acide de Brönsted.

**2.** Composition selon la revendication 1, **caractérisée par le fait que** A représente un groupe carbocyclique ou hétérocyclique, tel que défini dans la revendication 1, dans lequel au moins le cycle portant les deux fonctions aldéhydes vicinaux est un cycle aromatique.

**3.** Composition selon la revendication 2, **caractérisée par le fait que** l'$\alpha$-dialdéhyde de formule (I) est un orthophtalaldéhyde de formule (II)

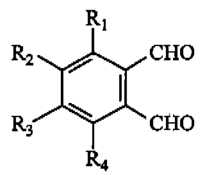

(II)

où $R^1$, $R^2$, $R^3$ et $R^4$ représentent chacun indépendamment un atome d'hydrogène, ou un substituant halogéno, alkyle en $C_{1-6}$, hydroxyle, alcoxy en $C_{1-6}$, thiol, acide carboxylique, nitro, sulfo ou hétérocyclique azoté.

**4.** Composition selon la revendication 3, **caractérisée par le fait que** $R^1$, $R^2$, $R^3$ et $R^4$ représentent tous un atome d'hydrogène.

**5.** Composition selon la revendication 2, **caractérisée par le fait que** l'$\alpha$-dialdéhyde est choisi parmi le 4,5-diméthoxy-orthophtalaidéhyde, le naphtalène-2,3-dialdéyde, l'anthracène-2,3-dialdéhyde et le thiophèna-2,3-dialdéhyde.

6. Composition selon l'une quelconque des revendications 1 à 5, **caractérisée par le fait que** le ou les sels d'ammonium d'un acide de Brönsted minéral sont choisis parmi le sulfate d'ammonium, l'hydrogénocarbonate d'ammonium et le phosphate d'ammonium.

7. Composition selon l'une quelconque des revendications 1 à 5, **caractérisée par le fait que** le ou les sels d'ammonium d'un acide de Bronsted organique sont choisis parmi les sels d'ammonium d'acides carboxyliques, les organosulfonates d'ammonium, les organosulfates d'ammonium, les organophosphates d'ammonium, les organophosphonates d'ammonium et les thlolates d'ammonium.

8. Composition selon la revendication 7, **caractérisé par le fait que** le ou les sels organiques d'ammonium sont choisis parmi l'hydrogénocarbonate d'ammonium et les carboxylates d'ammonium.

9. Composition selon la revendication 8, **caractérisé par le fait que** le sel organique d'ammonium est l'acétate d'ammonium.

10. Composition selon la revendication 7, **caractérisée par le fait que** le ou les sels organiques d'ammonium sont choisis parmi les sels d'ammonium de colorants organiques anioniques comportant au moins une fonction acide, les sels d'ammonium d'agents tensioactifs anioniques comportant au moins une fonction acide, les sels d'ammonium d'acides aminés, les polymères comportant des fonctions acides.

11. Composition selon l'une quelconque des revendications précédentes, **caractérisée par le fait qu'**elle a un pH compris entre 3 et 12, de préférence entre 6 et 11.

12. Composition selon l'une quelconque des revendications précédentes, **caractérisée par le fait que** la concentration en α-dialdéhyde de formule (I) est comprise entre 0,01 et 30 % en poids, de préférence entre 0,05 et 20 % en poids, rapporté au poids total de la composition.

13. Composition selon l'une quelconque des revendications précédentes, **caractérisée par le fait que** la concentration en sel d'ammonium d'un acide de Brönsted est comprise entre 0,01 et 30 % en poids, de préférence entre 0,05 et 20 % en poids, rapporté au poids total de la composition.

14. Procédé de teinture des fibres kératiniques comprenant les étapes suivantes consistant

   - à préparer, immédiatement avant emploi, une composition prête à l'emploi selon l'une quelconque des revendications précédentes,
   - à appliquer cette composition prête à l'emploi, préparée extemporanément, sur les fibres kératiniques,
   - à laisser la composition en contact avec les fibres kératiniques pendant un temps de pose suffisant pour obtenir la coloration recherchée, et
   - à rincer les fibres kératiniques.

15. Procédé de teinture des fibres kératiniques selon la revendication 14, **caractérisé par le fait que** l'on prépare la composition prête à l'emploi par mélange d'une première composition (a) contenant au moins un α-dialdéhyde de formule (I) et d'une deuxième composition (b) contenant au moins un sel d'ammonium d'un acide de Brönsted.

16. Procédé selon la revendication 14, **caractérisé par le fait que** l'on prépare la composition prête à l'emploi par mélange d'une première composition (c) contenant au moins un α-dialdéhyde de formule (I) et au moins un acide de Brönsted, et d'une deuxième composition (d) contenant de l'ammoniaque.

17. Procédé selon la revendication 14, **caractérisé par le fait que** l'on prépare la composition prête à l'emploi par mélange de trois compositions contenant respectivement au moins un α-dialdéhyde de formule (I), au moins un acide de Brönsted et de l'ammoniaque.

18. Procédé selon l'une quelconque des revendications 14 à 17, **caractérisé par le fait que** l'on laisse la composition prête à l'emploi en contact avec les fibres kératiniques pendant une période comprise entre 5 minutes et 1 heure, de préférence entre 5 et 30 minutes.

19. Procédé de teinture des fibres kératiniques comprenant les étapes suivantes consistant

• à appliquer sur les fibres kératiniques une première composition (a) contenant au moins un α-dialdéhyde de formule (I) et à laisser cette composition en contact avec les fibres kératiniques pendant un temps de pose compris entre 5 minutes et 1 heure,
• à procéder éventuellement à un rinçage des fibres kératiniques avec de l'eau.
• à appliquer sur les fibres kératiniques une deuxième composition (b) contenant au moins un sel d'ammonium d'un acide de Brônsted et à laisser cette composition en contact avec les fibres kératiniques pendant un temps de pose suffisant pour obtenir la coloration recherchée, puis
• à rincer les fibres kératiniques.

**20.** Procédé selon la revendication 19, **caractérisé par le fait que** l'on inverse l'ordre d'application des compositions (a) et (b).

**21.** Procédé de teinture des fibres kératiniques comprenant les étapes suivantes consistant

• à appliquer sur les fibres kératiniques une première composition (c) contenant au moins un α-dialdéhyde aromatique de formule (1) et au moins un acide de Bronsted, et à laisser cette composition en contact avec les fibres kératiniques pendant un temps de pose compris entre 5 minutes et 1 heure,
• à procéder éventuellement à un rinçage des fibres kératiniques avec de l'eau,
• à appliquer sur les fibres kératiniques une deuxième composition (d) contenant de l'ammoniaque et à laisser cette composition en contact avec les fibres kératiniques pendant un temps de pose suffisant pour obtenir la coloration recherchée, puis
• à rincer les fibres kératiniques.

**22.** Procédé selon la revendication 21, **caractérisé par le fait que** l'on inverse l'ordre d'application des compositions (c) et (d).

**23.** Procédé de teinture des fibres kératiniques comprenant les étapes suivantes consistant

• à appliquer sur les fibres kératiniques une première composition (e) contenant au moins un α-dialdéhyde aromatique de formule (I) et au moins de l'ammoniaque, et à laisser cette composition en contact avec les fibres kératiniques pendant un temps de pose compris entre 5 minutes et 1 heure, et de préférence entre 5 et 30 minutes,
• à procéder éventuellement à un rinçage des fibres kératiniques avec de l'eau,
• à appliquer sur les fibres kératiniques une deuxième composition (f) contenant un acide de Brönsted et à laisser cette composition en contact avec les fibres kératiniques pendant un temps de pose suffisant pour obtenir la coloration recherchée, puis
• à rincer les fibres kératiniques.

**24.** Procédé selon la revendication 23, **caractérisé par le fait que** l'on inverse l'ordre d'application des compositions (e) et (f).

**25.** Procédé selon l'une quelconque des revendications 14 à 24 **caractérisé par le fait que** l'on applique de la chaleur pendant le temps de pose de manière à augmenter la température jusqu'à une valeur au plus égale à 80 °C, de préférence au plus égale à 60 °C.

**26.** Procédé selon l'une quelconque des revendications 14 à 25 **caractérisé par le fait que** l'on effectue un post-traitement, consistant à appliquer une solution acide et à laisser cette solution en contact avec les fibres kératiniques, puis à rincer les fibres kératiniques.

**27.** Kit pour la teinture des fibres kératiniques, comprenant au moins deux compartiments, au moins l'un de ces compartiments contenant au moins un α-dialdéhyde de formule (1) (composition (a)) et au moins un autre compartiment contenant au moins un sel d'ammonium d'un acide de Brönsted (composition (b)).

**28.** Kit pour la teinture des fibres kératiniques, comprenant au moins deux compartiments, au moins l'un de ces compartiments contenant au moins un α-dialdéhyde de formule (I) et au moins un acide de Brönsted (composition (c)) et au moins un autre compartiment contenant de l'ammoniaque (composition (d)).

**29.** Kit pour la teinture des fibres kératiniques, comprenant au moins deux compartiments, au moins l'un de ces compartiments contenant au moins un α-dialdéhyde de formule (I) et de l'ammoniaque (composition (e)) et au moins

un autre compartiment contenant au moins un acide de Brönsted (composition (f)).

**30.** Kit pour la teinture des fibres kératiniques contenant au moins trois compartiments, au moins un premier contenant un α-dialdehyde de formule (1), au moins un deuxième contenant un acide de Bronsted et au moins un troisième contenant de l'ammoniaque.